# FASCICULE DE BREVET EUROPEEN

(11) **EP 4 432 546 B1**
(45) Date de publication et mention de la délivrance du brevet: **19.02.2025**
(21) Numéro de dépôt: 23315053.1
(22) Date de dépôt: 17.03.2023
(51) Int. Cl.: H02N 2/18, H10N 30/30, H10N 30/071, H10N 39/00, A61N 1/378

(54) **RÉCUPÉRATEUR D'ÉNERGIE À TRANSDUCTEUR PIÉZOÉLECTRIQUE, NOTAMMENT POUR L'ALIMENTATION D'UNE CAPSULE CARDIAQUE AUTONOME, AVEC STRUCTURE OSCILLANTE À GRADIENT DE RAIDEUR EN FLEXION**
ENERGIESAMMLER MIT PIEZOELEKTRISCHEM WANDLER, INSBESONDERE ZUR VERSORGUNG EINER AUTONOMEN KARDIOKAPSEL, MIT EINER OSZILLIERENDEN STRUKTUR MIT EINEM BIEGESTEIFIGKEITSGRADIENTEN
ENERGY HARVESTER WITH PIEZOELECTRIC TRANSDUCER, IN PARTICULAR FOR SUPPLYING AN AUTONOMOUS CARDIAC CAPSULE, WITH OSCILLATING STRUCTURE HAVING A GRADIENT OF FLEXURAL STIFFNESS

(43) Date de publication de la demande: 18.09.2024
(73) Titulaire: CAIRDAC, 92160 Antony (FR)
(72) Inventeur: Regnier, Willy, 91160 Longjumeau (FR); Nguyen-Dinh, An, 37520 La Riche (FR)
(74) Mandataire: Dupuis-Latour, Dominique

(56) Documents cités:
- EP-A1- 3 319 133
- EP-A1- 3 876 386
- WO-A1-2017/195014
- US-A1- 2015 008 792

## Description

### CONTEXTE DE L'INVENTION

### Domaine de l'invention

L'invention concerne les récupérateurs d'énergie, également dénommés "harvesters" ou "scavengers", qui recueillent l'énergie mécanique résultant de mouvements divers qu'ils subissent et convertissent cette énergie mécanique en énergie électrique.

Elle concerne plus spécifiquement les récupérateurs de type dit "PEH" (*Piezoelectric Energy Harvester*), qui utilisent comme transducteur mécano-électrique une lame piézoélectrique oscillante couplée à une masse mobile inertielle.

L'invention sera décrite plus particulièrement dans une application de ces récupérateurs d'énergie à des dispositifs médicaux autonomes, notamment les dispositifs de type capsule implantable autonome, en particulier ceux de ces dispositifs qui sont destinés à être implantés dans une cavité cardiaque.

Cette application, si elle est particulièrement avantageuse, ne doit toutefois pas être considérée comme limitative de l'invention, dont les enseignements peuvent être appliqués à de nombreux autres types de dispositifs autonomes incorporant un récupérateur d'énergie de type PEH, que ces dispositifs soient implantables ou non, qu'il s'agisse de dispositifs médicaux ou non.

### Description de la technique antérieure

Dans le domaine des implants médicaux, les récents progrès en matière de miniaturisation de dispositifs actifs et les progrès des sciences du vivant permettent dorénavant le développement d'une grande variété de systèmes miniaturisés implantables totalement autonomes, à des fins de surveillance, de diagnostic ou de traitement. Ces dispositifs mettent en œuvre des procédures d'implantation moins invasives, plus de confort, des performances accrues et souvent ouvrent l'accès à des nouveaux types de diagnostics et traitements.

Lorsqu'elle est appliquée au domaine des implants médicaux, l'invention concerne plus précisément ceux de ces implants qui incorporent un système d'autoalimentation comprenant un récupérateur d'énergie mécanique associé à un organe de stockage d'énergie intégré tel qu'une batterie rechargeable ou une capacité à hautes performances.

En effet, l'un des aspects critiques de ces dispositifs miniaturisés est celui de l'autonomie électrique. La durée de vie d'un tel implant étant d'environ 8 à 10 ans, compte tenu des très faibles dimensions il n'est pas possible d'utiliser une batterie conventionnelle, même à forte densité.

Le récupérateur d'énergie pallie cet inconvénient en recueillant l'énergie mécanique résultant des mouvements divers subis par le corps du dispositif implanté. Ces mouvements peuvent avoir pour origine un certain nombre de phénomènes se produisant par exemple au rythme des battements cardiaques tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit. L'énergie mécanique recueillie par le récupérateur est convertie en énergie électrique (tension ou courant) au moyen d'un transducteur mécanoélectrique approprié, pour assurer l'alimentation des divers circuits et capteurs du dispositif et la recharge de l'organe de stockage d'énergie. Ce système d'alimentation permet au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules *leadless",* pour les distinguer des électrodes ou capteurs disposés à l'extrémité distale d'une sonde (*lead*) parcourue sur toute sa longueur par un ou plusieurs conducteurs reliés à un générateur connecté à l'extrémité opposée, proximale.

L'invention n'est toutefois pas limitée à un type particulier de capsule, ni même d'implant leadless, et elle est applicable indifféremment à de nombreux autres types de dispositifs médicaux implantables, quelle que soit leur destination fonctionnelle, cardiaque ou autre, médicale ou non. Dans le cas d'une application cardiaque, la capsule leadless surveille en continu le rythme du patient et délivre si nécessaire au cœur des impulsions électriques de stimulation, de resynchronisation et/ou de défibrillation en cas de troubles du rythme détecté par la capsule. La capsule comprend par ailleurs divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Le WO 2019/001829 A1 (Cairdac) décrit un exemple d'une telle capsule leadless intracardiaque.

L'invention concerne plus précisément les capsules ou dispositifs implantables analogues dont le récupérateur d'énergie est du type PEH, c'est-à-dire utilisant un transducteur piézoélectrique et un ensemble pendulaire inertiel soumis aux sollicitations externes décrites plus haut. L'ensemble pendulaire inertiel comprend une masse mobile logée dans le corps de la capsule, dite "masse sismique" ou "masse inertielle", qui est entrainée au gré des mouvements de la capsule soumise en permanence aux diverses sollicitations externes décrites plus haut. Après chacune de ces sollicitations, la masse inertielle, qui est couplée à un élément élastiquement déformable, peut soit osciller à sa fréquence propre (si celle-ci est conçue pour être proche de la fréquence de la sollicitation) et s'amortir progressivement selon son mode d'amortissement (faible bande), soit se déformer sous l'effet de l'accélération en mode forcé (large bande).

L'énergie mécanique de l'oscillation est convertie en énergie électrique par un transducteur mécanoélectrique produisant un signal électrique. Ce transducteur mécanoélectrique peut en particulier être un transducteur piézoélectrique sollicité de façon cyclique en flexion de manière à engendrer au sein du matériau qui le constitue des charges électriques qui sont récupérées en surface du composant pour être utilisées par le système d'autoalimentation de la capsule leadless. Le transducteur piézoélectrique se présente le plus souvent sous la forme d'une lame encastrée à l'une de ses extrémités et couplée à la masse inertielle à son autre extrémité, qui est libre.

Le signal électrique en sortie du transducteur est délivré à un circuit de gestion de l'alimentation de la capsule, qui redresse et régule le signal électrique pour délivrer en sortie une tension ou un courant continus stabilisés permettant d'assurer l'alimentation des divers circuits électroniques et capteurs de la capsule, ainsi que la recharge de l'organe de stockage d'énergie.

Un tel récupérateur d'énergie de type PEH est notamment décrit dans le US 3,456,134 A (Ko) et dans le WO 2019/001829 A1 précité.

On notera que le terme de "lame" doit être entendu dans son sens le plus large, à savoir une bande mince et plate de forme allongée, étant entendu que la forme de cette bande n'est pas nécessairement rectangulaire ni son épaisseur constante (comme dans la description du mode de réalisation particulier qui sera donnée plus bas). Le terme de "lame" au sens de la présente invention recouvre ainsi des éléments pouvant présenter une largeur et/ou une épaisseur qui ne sont pas constantes en direction longitudinale, ainsi qu'éventuellement une déformabilité pouvant aller au-delà d'un unique degré de liberté en flexion.

Le problème de l'invention réside dans la recherche d'une simplification de l'architecture d'un PEH et, corrélativement, de l'architecture d'un dispositif médical implantable.autoalimenté par un tel PEH.

De façon générale, dans une optique de miniaturisation poussée et d'intégration du design d'un PEH, il est souhaitable de réduire le volume occupé non seulement par l'ensemble pendulaire proprement dit mais également par tous les composants et circuits annexes, de manière à libérer suffisamment d'espace pour le débattement de la lame oscillante à l'intérieur du dispositif implantable.

Dans cette même optique, il serait également souhaitable qu'un même élément de structure du PEH puisse assurer plusieurs fonctions, afin de réduire le volume global du système, en améliorer les performances, de faciliter l'industrialisation et de réduire le coût de fabrication.

Ainsi, le EP 3 876 386 A1 (Doliam), correspondant au US 2021 273587 A1 (Nguyen-Dinh et al.), propose de faire jouer à la lame oscillante du PEH, outre la fonction de production de charges électriques, une fonction de support mécanique pour l'unité de gestion de l'énergie (ci-après PMU, *Power Management Unit*) ainsi que pour la batterie tampon du PEH. Plus précisément, ce document propose de laminer un revêtement intercalaire isolant sur l'une des faces externes de la couche piézoélectrique de la lame oscillante du PZT, et de rapporter la puce du PMU ainsi que la batterie sur cet intercalaire externe. Ces composants sont fixés à l'intercalaire au moyen d'un point de colle ou d'un film adhésif, et leurs bornes de contact sont ensuite électriquement reliées par une brasure ou une colle conductrice à des pistes de liaison en surface de l'intercalaire.

Cet agencement permet d'augmenter la compacité d'ensemble du système et de gagner de la surface sur le(s) circuit(s) imprimé(s) (PCB) recevant les autres composants électriques et électroniques de l'implant.

Toutefois, du point de vue de l'industrialisation, compte tenu de la nature particulière de la lame sur laquelle la puce PMU et la batterie doivent être rapportées, cette technique est plus délicate à mettre en œuvre qu'un report sur un PCB conventionnel, dont le matériau et les caractéristiques physiques (notamment la rigidité et la robustesse) sont spécifiquement conçus pour recevoir des composants électroniques.

Un autre problème est celui de l'optimisation des caractéristiques proprement mécaniques de la lame oscillante de l'ensemble pendulaire, pour moduler la raideur en flexion de la lame du PZT de manière qu'elle présente un gradient non uniforme en direction longitudinale de la lame (c'est-à-dire dans la direction allant de l'extrémité distale libre à l'extrémité proximale encastrée de la lame). Avec un gradient de raideur, la contrainte de flexion appliquée à l'ensemble de la structure est plus importante à proximité de la zone d'encastrement, avec un maximum de 50 N/mm², et tend vers 0 N/mm² du côté opposé à proximité de la masse inertielle. Cette décroissance de la contrainte est progressive et non linéaire du fait de l'intégration de composants répartis de façon non homogène dans la structure silicium.

Par exemple, le WO 2017/195014 A1 (Vermon), correspondant au US 2017/0257040 A1 (Nguyen-Dinh et al.), propose de structurer la lame PZT avec une âme centrale ou *shim* composite incorporant des "piliers" ou des "parois" dont les diamètres ou les épaisseurs varient progressivement entre l'extrémité distale et l'extrémité proximale de la lame. Ces hétérogénéités contrôlées de la structure de l'âme centrale ont pour effet de faire varier corrélativement la raideur de la lame suivant la direction longitudinale, par exemple en la rendant i) plus flexible dans une région centrale et/ou distale, là où les déformations sont les plus importantes - ce qui permet d'accroître la quantité de charges électriques recueillies -, et ii) plus rigide dans une région proximale, au voisinage de l'encastrement - ce qui permet d'accroître la robustesse mécanique là où les contraintes de traction/compression sont les plus fortes.

Cette technique est toutefois, ici encore, difficile et coûteuse à mettre en œuvre du point de vue industriel compte tenu des dimensions extrêmement réduites d'une lame PZT lorsque celle-ci est destinée à alimenter un appareil qui doit être miniaturisé à l'extrême, surtout lorsqu'il s'agit de combiner dans un même élément (l'âme centrale) des matériaux aux propriétés mécaniques différentes pour y former les piliers ou les cloisons.

Les US 2015/008792 A1 et EP 3 319 133 A1 divulguent des récupérateurs de type PEH à cantilever MEMS, comportant des composants électroniques ou électriques intégrés de manière monolithique dans une lame flexible d'un substrat en Si, et leur fabrication.

### RÉSUMÉ DE L'INVENTION

Pour résoudre les différents problèmes et atteindre les buts exposés ci-dessus, l'invention propose un récupérateur d'énergie à transducteur piézoélectrique, PEH, comprenant un ensemble pendulaire soumis à des sollicitations externes appliquées au récupérateur. L'ensemble pendulaire comprend une lame élastiquement déformable en flexion, une monture de lame couplée avec encastrement à une extrémité proximale de la lame, et une masse inertielle montée à une extrémité distale, libre, de la lame. La lame est une lame piézoélectrique apte à convertir en un signal électrique oscillant une énergie mécanique produite par des oscillations de l'ensemble pendulaire. La lame comprend un structure sandwich flexible comportant une âme centrale, une couche piézoélectrique sur au moins l'une des faces de l'âme centrale, et au moins une électrode de surface sur une face externe de la couche piézoélectrique.

De façon caractéristique, selon un *premier aspect de l'invention* l'âme centrale de la structure sandwich flexible est en un matériau semi-conducteur apte à former un substrat de circuit intégré, et au moins une partie des composants d'une unité électrique ou électronique sont intégrés sous forme monolithique dans le substrat en matériau semi-conducteur de l'âme centrale de la structure sandwich flexible.

Selon diverses formes de réalisation avantageuses :
- l'unité électrique ou électronique comprend des composants d'une unité de gestion d'alimentation, PMU, intégrés dans l'âme centrale, la PMU étant apte à redresser et réguler le signal électrique oscillant pour délivrer en sortie une tension continue ou un courant continu stabilisés ;
- l'unité électrique ou électronique comprend des composants *solid-state* d'un organe de stockage d'énergie intégrés dans l'âme centrale, l'organe de stockage d'énergie étant apte à être rechargé à partir du signal électrique oscillant produit par les oscillations de l'ensemble pendulaire ;
- l'unité électrique ou électronique comprend des composants d'un processeur numérique intégrés dans l'âme centrale ;
- l'âme centrale comporte en outre, au voisinage de l'extrémité proximale dans une région non couverte par une couche piézoélectrique, des plages de connexion électriquement reliées à des composants du processeur numérique intégrés dans l'âme centrale ; et/ou
- au moins une partie des composants de l'unité électrique ou électronique sont intégrés sous forme monolithique dans une région distale de l'âme centrale à proximité, ou au niveau, de la masse inertielle.

L'invention a également pour objet un procédé de fabrication d'un tel PEH, comprenant les étapes suivantes :
a) obtention d'une âme centrale en un matériau semi-conducteur apte à former un substrat de circuit intégré ;
b) intégration sous forme monolithique, dans le substrat en matériau semi-conducteur de l'âme centrale, d'au moins une partie des composants d'une unité électrique ou électronique ;
c) dépôt, sur au moins l'une des faces de l'âme centrale, d'une couche piézoélectrique ; et
d) dépôt d'au moins une électrode de surface sur une face externe de la couche piézoélectrique.

Selon diverses modalités avantageuses de mise en œuvre :
- l'étape b) comprend l'intégration sous forme monolithique dans le substrat en matériau semi-conducteur de l'âme centrale de composants d'une unité de gestion d'alimentation, PMU, de composants solid-state d'un organe de stockage d'énergie, et/ou de composants d'un processeur numérique ;
- le procédé comprend en outre des étapes ultérieures de : e) montage d'une masse inertielle à une extrémité distale, libre, de la lame ; et f) couplage avec encastrement d'une extrémité proximale de la lame à une monture de lame ;
- en particulier, l'étape e) comprend le collage de demi-masses directement sur les faces externes respectives de la couche piézoélectrique, et/ou l'étape f) comprend le collage de deux éléments de monture directement sur les faces externes respectives de la couche piézoélectrique.

Selon un *second aspect de l'invention,* l'âme centrale de la structure sandwich flexible est en un matériau semi-conducteur apte à former un substrat de circuit intégré, le substrat en matériau semi-conducteur de l'âme centrale de la structure sandwich flexible comporte des structures intégrées monolithiques, et l'agencement, sur l'étendue du substrat de l'âme centrale, desdites structures intégrées monolithiques forme en direction longitudinale une pluralité de zones successives ayant des coefficients de raideur en flexion de la lame piézoélectrique différents d'une zone à l'autre.

Selon diverses formes de réalisation avantageuses :
- lesdites structures intégrées monolithiques sont des composants électriques ou électroniques intégrés ;
- lesdits composants électriques ou électroniques intégrés sont des composants électroniques d'une unité de gestion d'alimentation, PMU, intégrée à l'âme centrale de la structure sandwich flexible, et/ou des composants solid-state d'un organe de stockage d'énergie intégré à l'âme centrale de la structure sandwich flexible, et/ou des composants électroniques d'un processeur numérique intégré à l'âme centrale de la structure sandwich flexible ;
- la pluralité de zones successives comprend, en direction longitudinale, au moins une zone centrale et des zones distale et proximale attenantes, et dans lequel la raideur en flexion de la zone centrale est inférieure à la raideur en flexion des zones distale et proximale attenantes ;
- en particulier, la zone distale est au moins partiellement située dans une région de la masse inertielle, et/ou la zone proximale est au moins partiellement située dans une région de la monture de lame ;

L'invention a également pour objet un procédé de fabrication d'un tel PEH, comprenant les étapes suivantes :
a) obtention d'une âme centrale en un matériau semi-conducteur apte à former un substrat de circuit intégré ;
b) intégration de structures intégrées monolithiques dans le substrat en matériau semi-conducteur de l'âme centrale, où l'agencement, sur l'étendue du substrat de l'âme centrale, desdites structures intégrées monolithiques forme en direction longitudinale une pluralité de zones successives ayant des coefficients de raideur en flexion de la lame piézoélectrique différents d'une zone à l'autre ;
c) dépôt, sur au moins l'une des faces de l'âme centrale, d'une couche piézoélectrique ; et
d) dépôt d'au moins une électrode de surface sur une face externe de la couche piézoélectrique.

Selon diverses modalités avantageuses de mise en œuvre :
- l'étape b) comprend l'intégration sous forme monolithique de composants électriques ou électroniques ;
- lesdits composants électriques ou électroniques intégrés à l'étape b) sont des composants électroniques d'une unité de gestion d'alimentation, PMU, intégrée à l'âme centrale de la structure sandwich flexible, et/ou des composants *solid-state* d'un organe de stockage d'énergie intégré à l'âme centrale de la structure sandwich flexible, et/ou des composants électroniques d'un processeur numérique intégré à l'âme centrale de la structure sandwich flexible ;
- le procédé comprend en outre des étapes ultérieures de : e) montage d'une masse inertielle à une extrémité distale, libre, de la lame ; et f) couplage avec encastrement d'une extrémité proximale de la lame à une monture de lame ;
- en particulier, l'étape e) comprend le collage de demi-masses directement sur les faces externes respectives de la couche piézoélectrique, et/ou l'étape f) comprend le collage de deux éléments de monture directement sur les faces externes respectives de la couche piézoélectrique.

L'invention a également pour objet un dispositif autonome logeant, dans un corps de dispositif, un ensemble électronique, un organe de stockage d'énergie, et un PEH tel que ci-dessus, pour l'alimentation de l'ensemble électronique et/ou la recharge de l'organe de stockage d'énergie.

Le dispositif autonome peut en particulier être un dispositif médical actif de type capsule autonome implantable comprenant un corps de capsule avec un élément d'ancrage à une paroi d'un organe d'un patient, les sollicitations externes auxquelles est soumis l'ensemble pendulaire du module de récupération d'énergie étant des sollicitations appliquées au corps de capsule sous l'effet de mouvements de ladite paroi et/ou de variations de débit d'un flux dans le milieu environnant.

### DESCRIPTION SOMMAIRE DES DESSINS

On va maintenant décrire un exemple de réalisation de la présente invention en référence aux dessins annexés, où les mêmes références désignent d'une figure à l'autre des éléments identiques ou fonctionnellement semblables.
La Figure 1 illustre des dispositifs médicaux de type capsule leadless dans leur environnement, avec divers exemples de sites d'implantation dans, sur ou à proximité du cœur d'un patient.
La Figure 2 illustre une capsule leadless implantée au fond du ventricule droit d'un patient.
La Figure 3 montre isolément un ensemble pendulaire de type connu, avec un transducteur piézoélectrique en forme de lame allongée encastrée à une extrémité et supportant une masse inertielle à son extrémité opposée.
La Figure 4 présente sous forme schématique les principaux blocs fonctionnels constitutifs d'une capsule leadless.
La Figure 5 est une vue en coupe montrant, avec une échelle des épaisseurs volontairement exagérée, la structure des différentes couches d'un transducteur piézoélectrique bimorphe utilisable avec la présente invention.
La Figure 6 est une vue en coupe montrant, avec une échelle des épaisseurs volontairement exagérée, la structure des différentes couches d'un transducteur piézoélectrique bimorphe selon l'invention, dans un premier mode de réalisation où l'âme centrale de la lame piézoélectrique intègre des composants électroniques monolithiques.
La Figure 7 illustre, de façon générale, la manière d'obtenir en direction longitudinale un gradient de raideur variable sur l'étendue de la lame piézoélectrique, selon les enseignements de l'invention.
La Figure 8 est une vue éclatée d'un transducteur piézoélectrique selon l'invention tel que celui de la Figure 6 dans un mode de réalisation intégrant des composants monolithiques, cette vue montrant séparément les différents éléments constitutifs du transducteur.
La Figure 9 est une vue perspective du transducteur piézoélectrique de la Figure 8, dans une configuration finale assemblée.
La Figure 10 montre isolément, en perspective, l'âme centrale de la lame piézoélectrique du transducteur de la Figure 8.
La Figure 11 est un détail agrandi, en coupe, de la lame du transducteur piézoélectrique des Figures 8 à 10, cette vue montrant la manière de réaliser les interconnexions au sein des différentes couches de l'âme centrale, et entre ces couches et les surfaces internes des couches piézoélectriques de la lame.
La Figure 12 illustre la manière dont se répartit le gradient de raideur variable sur l'étendue de la lame piézoélectrique du mode de réalisation de la Figure 11.
La Figure 13 est une courbe donnant la caractéristique de variation du gradient de raideur en fonction de la distance pour la lame piézoélectrique illustrée Figure 12.
La Figure 14 est homologue de la Figure 11, pour une variante de réalisation du transducteur.
La Figure 15 est un organigramme présentant les différentes étapes d'un processus de fabrication d'un PEH selon l'invention.

### DESCRIPTION DÉTAILLÉE DE MODES DE RÉALISATION PRÉFÉRENTIELS DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est donnée qu'à titre d'exemple de réalisation et n'est pas limitative de l'invention, dont les enseignements peuvent être appliqués à de nombreux autre types de dispositifs autonomes incorporant un récupérateur d'énergie de type PEH, que ces dispositifs soient implantables ou non, qu'il s'agisse de dispositifs médicaux ou non.

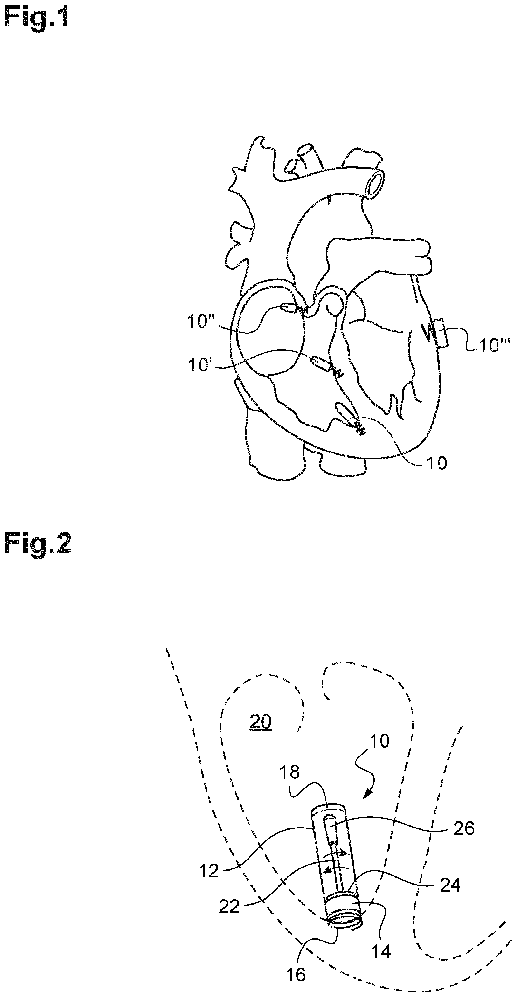

Sur la Figure 1, on a représenté diverses possibilités de sites d'implantation d'un dispositif de type leadless, dans une application à la stimulation cardiaque. Ainsi, la capsule 10 est implantée à l'intérieur d'une cavité du myocarde (implant endocavitaire), par exemple à l'apex du ventricule droit. En variante, la capsule peut être également implantée sur le septum interventriculaire droit, comme en 10', ou encore sur une paroi auriculaire, comme illustré en 10". Le dispositif peut également être une capsule épicardique placée sur une région externe du myocarde, comme illustré en 10"'.

Dans chaque cas, la capsule leadless est fixée à la paroi cardiaque au moyen d'un système d'ancrage saillant pénétrant dans le tissu cardiaque pour le maintien sur le site d'implantation. D'autres systèmes d'ancrage sont utilisables, et ne modifient en aucune façon la mise en œuvre de la présente invention.

La capsule 10 se présente sous forme extérieure d'un implant avec un corps tubulaire allongé 12 enfermant les divers circuits électroniques et d'alimentation de la capsule ainsi qu'un récupérateur d'énergie à ensemble pendulaire. Les dimensions typiques des capsules connues sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en œuvre de la présente invention. L'extrémité opposée (proximale) 18 de la capsule 10 est une extrémité libre, qui est seulement pourvue de moyens (non représentés) de liaison temporaire à un cathéter-guide ou autre accessoire d'implantation utilisé pour la mise en place ou l'explantation de la capsule, qui est ensuite désolidarisé de cette dernière.

Dans l'exemple illustré Figure 2, la capsule leadless 10 est un implant endocavitaire implanté à l'intérieur d'une cavité 20 du myocarde, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde, ces différents modes d'implantation ne modifiant en aucune façon la mise en œuvre de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. ·Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques.

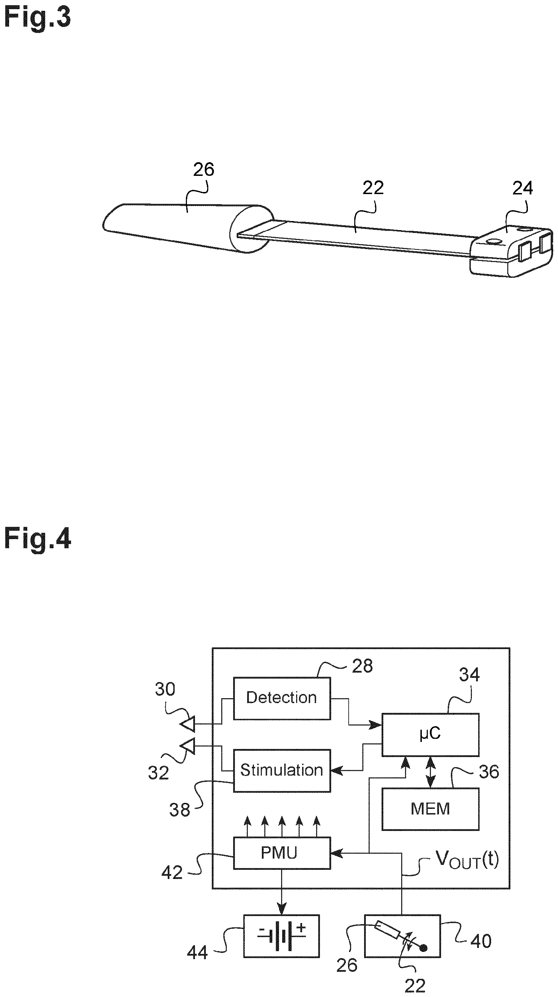

L'ensemble pendulaire, illustré isolément Figure 3, est constitué d'une lame piézoélectrique 22 encastrée en 24 à l'une de ses extrémités et dont l'extrémité opposée, libre, est couplée à une masse inertielle mobile 26. La lame piézoélectrique 22 est une lame flexible élastiquement déformable qui constitue avec la masse inertielle 26 un système pendulaire de type masse-ressort. Du fait de son inertie, la masse 26 soumet la lame 22 à une déformation de type vibratoire de part et d'autre d'une position neutre ou non déformée correspondant à une position stable de repos en l'absence de toute sollicitation. Les dimensions typiques minimales des lames de transducteur piézoélectrique des dispositifs connus de ce type sont de l'ordre de 25 mm de long pour une largeur de 5 mm environ.

De fait, pour ce qui est de son comportement mécanique, cet ensemble peut être assimilé à une structure de type "poutre encastrée-libre", présentant une fréquence d'oscillation propre qui est ici la fréquence sur laquelle oscille le système masse-ressort. On notera que cette fréquence d'oscillation propre, typiquement de l'ordre de quelques dizaines de hertz, est notablement supérieure à la fréquence des sollicitations cycliques externes qui correspondent à la fréquence des battements cardiaques (quelques hertz tout au plus). Ainsi, à chaque contraction cardiaque la masse inertielle (ou autre organe mécanique fonctionnellement analogue) sera sollicitée avec une amplitude plus ou moins importante, puis le système pendulaire oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

La lame 22 assure en outre, par effet piézoélectrique, une fonction de transducteur mécanoélectrique permettant de convertir en charges électriques la contrainte mécanique de flexion qui lui est appliquée. Ces charges sont collectées par des électrodes à la surface de la lame de manière à produire un signal électrique qui, après redressement, stabilisation et filtrage, alimentera les divers circuits électroniques de la capsule.

La Figure 4 est un synoptique des divers circuits électriques et électroniques intégrés à la capsule leadless, présenté sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule. Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer au système d'électrodes 30, 32 des impulsions de stimulation du myocarde.

Il est par ailleurs prévu un circuit récupérateur d'énergie ou PEH 40, constitué par l'ensemble pendulaire formé par la lame piézoélectrique 22 et la masse inertielle 26 décrits plus haut en référence aux Figures 2 et 3. Comme la lame piézoélectrique 22 assure aussi une fonction de transducteur mécano-électrique, elle convertit en charges électriques les sollicitations mécaniques subies et produit un signal électrique variable V_{OUT}(t), qui est un signal alternatif oscillant à la fréquence d'oscillation libre de l'ensemble pendulaire lame 22/masse 26, et au rythme des battements successifs du myocarde auquel la capsule est couplée.

Le signal électrique variable V_{OUT}(t) est délivré à un circuit de gestion de l'énergie ou PMU 42. Le PMU 42 redresse et régule le signal V_{OUT}(t) de manière à produire en sortie une tension ou un courant continus stabilisés servant à l'alimentation des divers circuits électroniques et à la recharge de la batterie intégrée 44.

D'autre part, la lame est avantageusement une lame de type bimorphe, c'est-à-dire capable de générer de l'énergie sur ses deux faces lorsqu'elle est soumise à une déformation. Ces propriétés de transduction sont typiques d'un matériau piézoélectrique tel que les céramiques PZT, titano-zirconate de plomb, ou les monocristaux de type PMN-PT, titanate de baryum ou niobate de lithium.

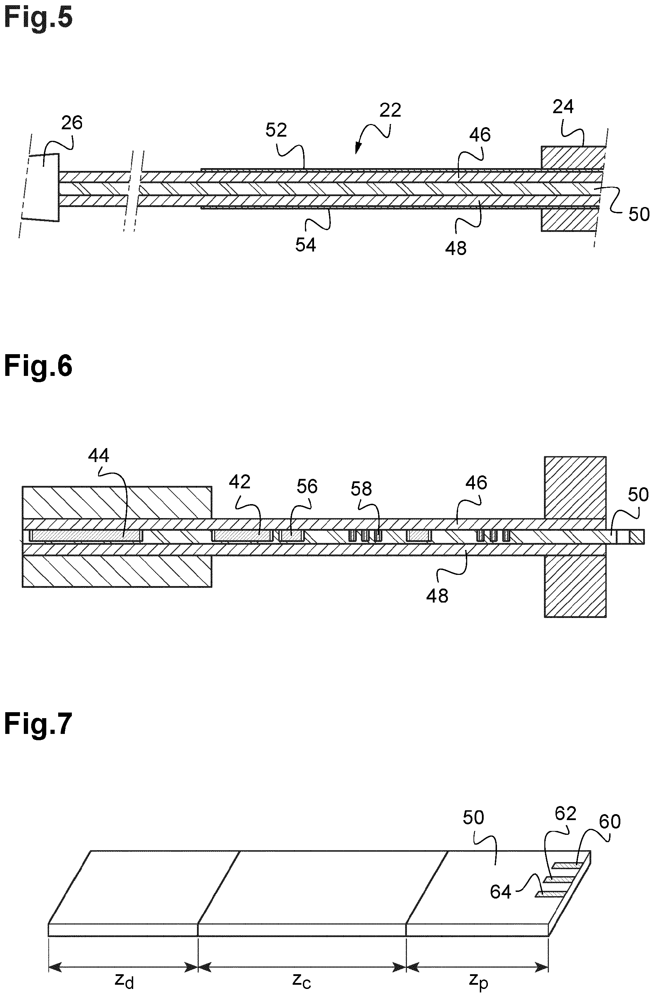

La Figure 5 illustre de façon schématique, avec une échelle des épaisseurs volontairement exagérée, la structure des différentes couches d'une telle lame piézoélectrique bimorphe.

La lame 22 bimorphe comprend deux couches 46, 48 de matériau céramique piézoélectrique, par exemple une céramique PZT, déposées sur chacune des faces opposées d'une âme centrale ou *shim 50.*

L'âme centrale 50 est généralement réalisée en un matériau conducteur tel que le laiton, mais il a été également proposé de la réaliser en un matériau isolant, avec dans ce cas des ponts de contact permettant de shunter les électrodes internes des couches piézoélectriques.

La structure bimorphe illustrée correspond en fait à l'association de deux structures unimorphes placées dos à dos, avec mise en commun de l'âme centrale supportant le matériau piézoélectrique (ce matériau étant supporté sur une seule des faces de l'âme centrale dans le cas d'une structure unimorphe, et sur l'une et l'autre face dans le cas d'une structure bimorphe).

De fait, tout ce qui sera exposé par la suite dans les exemples décrits, qui montrent une structure bimorphe, est transposable aussi bien à une structure unimorphe, en conservant les mêmes agencements de composants et éléments du transducteur, mais d'un seul côté de l'âme centrale au lieu de l'être des deux côtés, les enseignements de l'invention restant appliqués de la même manière.

Dans une structure conventionnelle telle que celle de la Figure 5 où l'âme 50 est en matériau conducteur, typiquement en laiton, les charges produites par la déformation du matériau des couches piézoélectriques 46, 48 sont recueillies, côté externe, par des électrodes de surface 52, 54 déposées sur des faces opposées de la lame, et, côté interne, par l'âme centrale 50 conductrice.

Dans une version simplifiée illustrée ici, la géométrie de la lame est de forme rectangulaire, pour une largeur typique de 5 mm maximum et une longueur de 35 mm maximum. L'épaisseur des couches piézoélectriques 46, 48 disposées de part et d'autre de l'âme centrale en silicium est typiquement de 300 µm.

Comme cela est décrit dans le EP 3 930 014 A1 (Cairdac), afin de renforcer la fiabilité des lames 22, 22', il est possible de donner à celles-ci, sur tout ou partie de leur longueur, une forme en plan trapézoïdale avec une décroissance (linéaire ou exponentielle) de la largeur pour mieux répartir les contraintes le long de la lame, ces contraintes étant plus fortes à proximité et au niveau de l'encastrement 24, et nulles au niveau de la masse inertielle 26. De plus, la forme trapézoïdale permet d'ajuster la fréquence de résonance de l'ensemble en fonction de la géométrie du trapèze, tout en maximisant l'amplitude de déplacement de la masse du fait que l'extrémité libre est plus étroite que l'extrémité encastrée.

La Figure 6 est homologue de la Figure 5, pour un transducteur piézoélectrique agencé selon un mode de réalisation particulier de l'invention, avec intégration de composants monolithiques.

Comme indiqué plus haut, dans les transducteurs piézoélectriques connus, l'âme centrale est généralement en un matériau métallique tel que le laiton, en particulier pour permettre une prise de connexion directe avec la couche piézoélectrique sus-jacente, et ainsi la captation des charges produites par la déformation du matériau piézoélectrique.

Selon l'invention, cette âme centrale n'est pas en un matériau métallique, ni non plus en un matériau isolant, mais en un matériau semi-conducteur, et cette âme centrale semi-conductrice forme notamment un substrat dans lequel peuvent être intégrées des structures monolithiques.

Ces structures monolithiques peuvent avantageusement (mais non nécessairement) comprendre des composants électriques ou électroniques intégrés.

Ces composants électriques ou électroniques peuvent avantageusement (mais non nécessairement) comprendre le circuit de gestion de l'énergie PMU 42 et/ou la batterie intégrée 44 dont les rôles ont été exposés plus haut en référence à la Figure 4.

D'autres composants, électroniques ou électriques, actifs ou passifs, référencés 56, 58, ..., peuvent être également intégrés au substrat de l'âme 50 sous forme monolithique.

Ces diverses structures monolithiques peuvent être réalisées au moyen de techniques conventionnelles de fabrication de micropuces par gravure, épitaxie, masquage, dépôt de couches, etc., bien connues en elles-mêmes et qui ne seront pas décrites en détail.

Le matériau semi-conducteur est avantageusement le silicium, compte tenu de ses bonnes propriétés mécaniques et de la possibilité d'y réaliser diverses structures intégrées monolithiques par des techniques aisées à mettre en œuvre, et ceci sur une épaisseur très réduite, typiquement de 10 à 20 µm au plus. Il est en effet nécessaire de conserver à l'âme centrale 50, et donc à la lame du transducteur piézoélectrique, une flexibilité suffisante pour que les couches de matériau piézoélectrique puissent se déformer et produire les charges électriques de la manière souhaitée.

Par commodité, on envisagera par la suite une âme centrale en silicium, mais le choix de ce matériau n'est aucunement limitatif de l'invention et d'autres matériaux semi-conducteurs peuvent être utilisés aussi bien pour mettre en œuvre les enseignements de l'invention, comme on le comprendra à la suite à la lecture de la description des différents modes de mise en œuvre exposés ci-après.

Selon *un premier aspect de l'invention,* les structures intégrées monolithiques de l'âme centrale de la lame sont des composants d'unités électriques ou électroniques intégrées associées au PEH et/ou au dispositif alimenté par ce PEH (vu autrement, selon l'invention, le substrat d'une micropuce du PEH supporte une couche de matériau piézoélectrique sur l'une au moins de ses faces).

Dans une première mise en œuvre, particulièrement avantageuse, les structures intégrées monolithiques sont des composants d'un PMU directement couplé aux électrodes de recueil des charges de la lame piézoélectrique, c'est-à-dire un PMU tel que celui référencé 42 sur la Figure 4, qui reçoit et traite les charges produites par la déformation du matériau piézoélectrique.

La gestion de l'énergie produite par la lame piézoélectrique et la délivrance d'une tension ou d'un courant réguliers d'alimentation par le PMU peuvent être ainsi au moins partiellement, et de préférence totalement, intégrées à l'ensemble pendulaire i) sans qu'il soit nécessaire de reporter aucun composant additionnel sur la lame (comme dans le WO 2017/195014 A1 précité où la puce du PMU est un composant rapporté, supporté par la lame), et ii) sans liaison des électrodes de la lame à un circuit distant situé sur un PCB agencé dans le dispositif au voisinage du PEH mais séparément de ce dernier (comme dans la configuration conventionnelle telle que celle du WO 2019/001829 A1 précité).

Le PEH selon l'invention se présente alors sous la simple forme d'un ensemble pendulaire (lame piézoélectrique + masse inertielle + monture de lame) avec des plages ou *pads* de sortie délivrant une tension ou un courant régulés directement utilisables pour l'alimentation d'un dispositif associé à ce PEH. Le dispositif ne nécessite dès lors aucun PMU ou circuit propre de régulation spécifiquement adapté au PEH utilisé.

Dans une deuxième mise en œuvre, en variante ou - avantageusement - en complément de la précédente, certaines des structures intégrées monolithiques sont des composants d'une batterie *solid-state* alimentée à partir des charges générées par les oscillations de la lame piézoélectrique. Les batteries *solid-state* sont des composants bien connus en eux-mêmes, notamment les batteries lithium *solid-state* qui peuvent avantageusement remplacer les batteries liquides lithium-ion ou lithium-polymère. Les batteries *solid-state* sont réalisées par dépôt de fines couches de lithium sur un substrat silicium, avec des procédés en eux-mêmes connus qui ne seront pas décrits plus en détail.

Dans une troisième mise en œuvre, en variante ou en complément des précédentes, les structures intégrées monolithiques sont des composants du dispositif alimenté par le PEH selon l'invention, notamment :
- des composants d'un processeur numérique (tel que le microcontrôleur 34 de la Figure 4),
- et/ou des composants d'un circuit de stimulation ou de détection (les circuits 28 et 38 de la Figure 4),
- et/ou des composants actifs (notamment des diodes) ou passifs (résistances, condensateurs, etc.).

Dans tous les cas, l'intégration de composants électriques ou électroniques monolithiques dans l'âme centrale de la lame du PZT contribue à accroître la compacité d'ensemble du dispositif et à simplifier la réalisation industrielle de celui-ci.

Selon *un second aspect de l'invention,* les structures intégrées monolithiques servent à appliquer à la lame un gradient de raideur en flexion différencié en direction longitudinale (c'est-à-dire entre l'extrémité distale libre et l'extrémité proximale encastrée).

Plus précisément, l'agencement différencié des structures intégrées monolithiques formées dans le substrat en silicium de l'âme centrale permet de définir une pluralité de zones successives présentant des coefficients de raideur en flexion de la lame qui sont différents d'une zone à l'autre.

Ainsi, comme illustré sur la Figure 7 qui montre isolément la lame piézoélectrique en perspective, il est possible de prévoir :
- une zone centrale z_{c} plus flexible, là où les contraintes de flexion sont plus importantes, de manière à favoriser la production de charges électriques dans le matériau piézoélectrique dans cette zone ; et/ou
- des zones d'extrémité distale z_{d} ou proximale zₚ plus rigides, là où les contraintes de flexion sont moindres, mais où il est souhaitable d'augmenter la rigidité mécanique de la lame pour supporter la masse inertielle (côté distal) et assurer un encastrement robuste de la lame dans sa monture (côté proximal).

Les structures intégrées monolithiques agencées dans l'âme centrale 50 pour définir le gradient de raideur en flexion différencié selon les zones peuvent être des composants électriques ou électroniques tels que ceux exposés plus haut à propos du premier mode de réalisation.

Mais il ne s'agit là que d'une possibilité, et tout ou partie des structures intégrées monolithiques définissant le gradient de raideur peuvent être des structures non électriquement fonctionnelles, n'ayant en elles-mêmes qu'un rôle mécanique.

Les longueurs de chacune des trois zones z_{d}, z_{c} et zₚ sont typiquement inférieures à 15 mm, avec une largeur typiquement inférieure à 5 mm, ces valeurs n'étant bien entendu données ici qu'à titre illustratif.

On notera incidemment que l'intégration de la batterie 44 dans une région distale de l'âme centrale 50 à proximité, ou au niveau, de la masse inertielle 26 permet d'ajouter localement la masse propre de la batterie à celle de la masse inertielle, favorisant ainsi le comportement en oscillation de l'ensemble pendulaire.

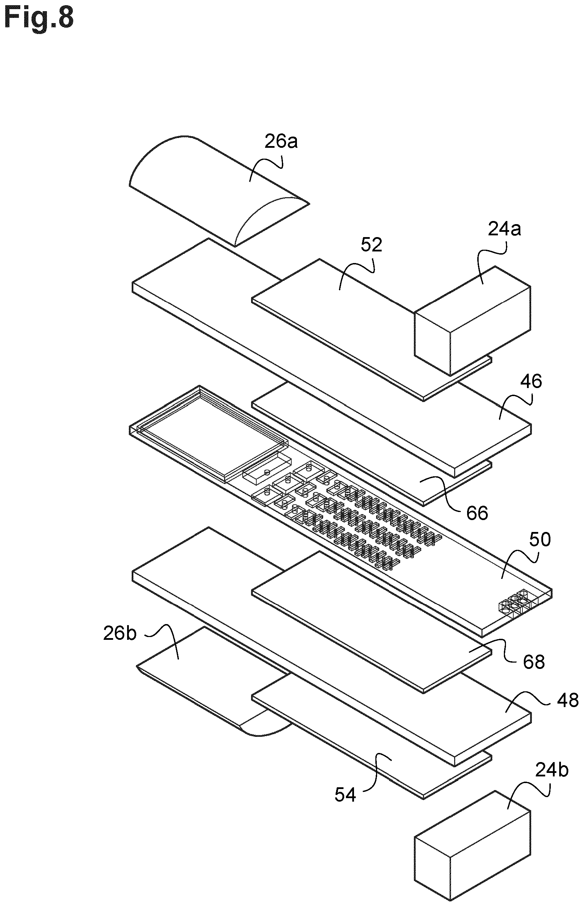

Les Figures 8 à 11 illustrent plus en détail la structure interne d'un PEH réalisé selon le premier aspect de l'invention indiqué plus haut, c'est-à-dire avec intégration monolithique d'éléments ayant une fonctionnalité électrique ou électronique.

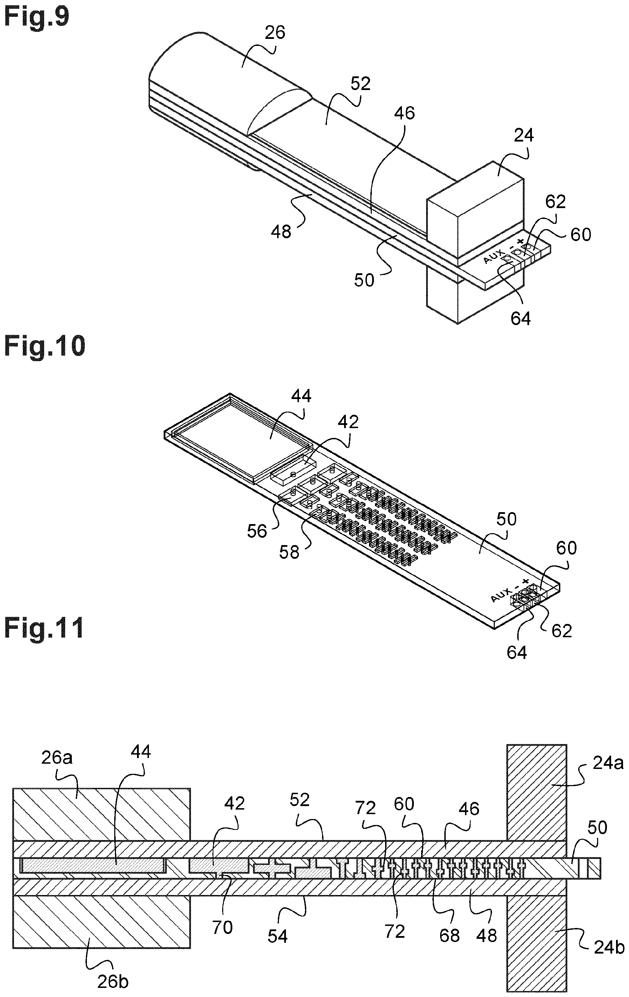

Comme on peut le voir sur la vue éclatée de la Figure 8 et sur la vue en configuration finale de la Figure 9, le PEH selon l'invention comprend l'âme centrale 50 avec, de part et d'autre, une couche de matériau piézoélectrique 46, 48 (dans le cas d'une lame de PEH de type bimorphe ; dans le cas d'une lame unimorphe la couche piézoélectrique ne serait présente que sur l'une des faces de l'âme centrale 50).

Sur leur face extérieure, les couches piézoélectriques 46, 48 comportent une électrode conductrice respective 52, 54 permettant de recueillir les charges électriques.

Sur leur face intérieure, c'est-à-dire la face tournée vers l'âme centrale 50, les couches piézoélectriques 46, 48 sont recouvertes d'une couche intercalaire isolante, respectivement 66, 68, réalisée par exemple par dépôt de parylène. Le parylène est un polymère qui peut être déposé sous vide et recevoir une couche de surface conductrice permettant de réaliser diverses connexions électriques entre des points prédéfinis de la couche, correspondant à des zones en vis-à-vis de l'âme centrale 50 nécessitant une prise de contact, sélectivement sur chacun des éléments électriques ou électroniques intégrés à l'âme centrale 50.

Comme on peut le voir sur la Figure 10 qui montre isolément l'âme centrale 50, celle-ci intègre un certain nombre d'éléments électriques ou électroniques tels que : batterie *solid-state* 44, PMU 42, puces diverses 56 de circuit numérique ou analogique, composants actifs ou passifs 58, etc. Les interconnexions entre ces divers éléments, et avec les électrodes internes formées sur les couches intercalaires 66, 68, sont effectuées au moyen de vias, ou bien directement contre une zone conductrice de la couche intercalaire 66, 68.

Par exemple, comme on peut le voir sur la vue en coupe de la Figure 11, le PMU 42 est en liaison électriquè directe avec une zone conductrice de la surface interne de la couche 46, de même que l'une des bornes de la batterie 44, tandis qu'une autre borne de ce même PMU est reliée à un point de contact spécifique par un via tel que celui illustré en 70. D'autres vias, tels que ceux illustrés en 72, permettent d'assurer des interconnexions entre les diverses couches du substrat semi-conducteur de l'âme centrale 50, ainsi qu'éventuellement avec des zones conductrices de la face interne des couches intercalaires.46, 48.

Dans un mode de réalisation où l'âme interne intègre tous les composants nécessaires au circuit d'alimentation d'un dispositif électronique, l'extrémité proximale peut être terminée par des plages de connexion 60, 62, 64, par exemple une plage positive (+) 60, une plage négative (-) 62 et une plage auxiliaire (AUX) 64, le dispositif électronique alimenté par le PEH de l'invention étant simplement relié à ces bornes comme il le serait à une simple pile ou batterie.

Dans cette configuration, le PEH incorpore, de façon caractéristique de l'invention, la totalité des organes de recueil de l'énergie, de stockage intermédiaire (par la batterie 44) et de régulation (par le PMU 42).

La voie auxiliaire peut servir notamment pour une recharge externe de la batterie lorsque le dispositif est dans un état de stockage prolongé avant implantation, comme cela est décrit par exemple dans les EP 4 019 083 A1, EP 4 015 034 A1 et EP 4 015 036 A1 (Cairdac).

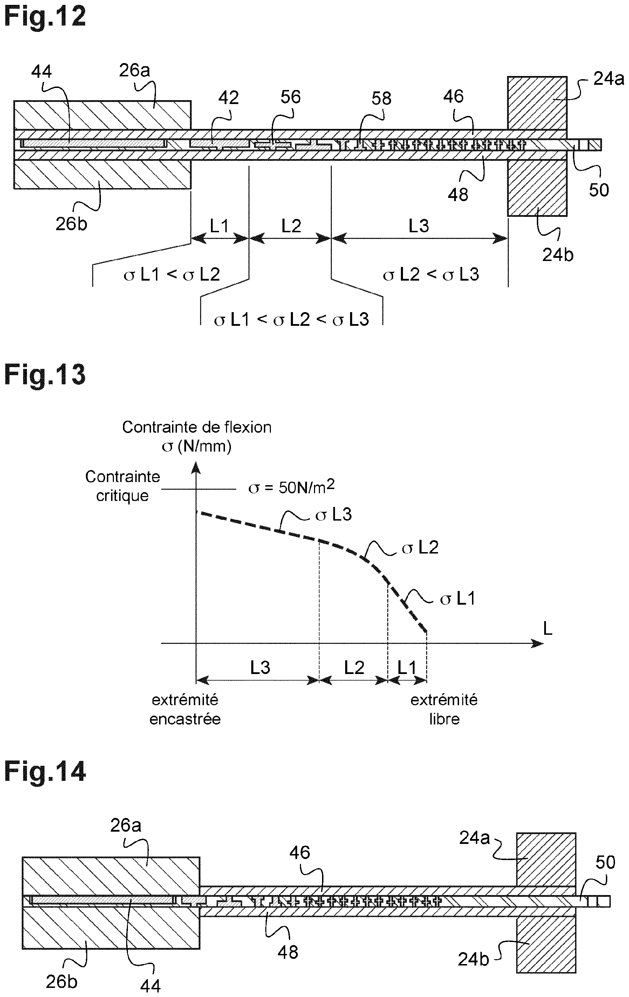

La Figure 12 illustre la manière dont se répartit le gradient de raideur variable sur l'étendue de la lame piézoélectrique du mode de réalisation de la Figure 11, et la Figure 13 est une courbe donnant la caractéristique de variation du gradient de raideur en fonction de la distance pour cette lame piézoélectrique.

Sur l'ensemble constitué par l'âme centrale en silicium 50, les couches PZT 46, 48, et les électrodes de surface 52, 54, la contrainte de flexion σ exercée décroît régulièrement avec la distance (zones successives L3, L2 et L1) au fur et à mesure que l'on s'éloigne du point d'encastrement de la monture 24. Dans le mode de réalisation illustré, la contrainte typique de flexion ne dépasse pas une valeur critique de 50 N/mm².

Ainsi, l'intégration de composants répartis de façon non homogène dans la structure de l'âme centrale en silicium 50 introduit un gradient de raideur tel que la contrainte de flexion appliquée à l'ensemble de la structure diminue de façon progressive et non linéaire ; cette contrainte est plus importante à proximité de la zone d'encastrement, avec un maximum admissible de 50 N/mm², et tend vers 0 N/mm² du côté opposé, à proximité des masses inertielles.

La Figure 14 est homologue de la Figure 11, pour une variante de réalisation du transducteur.

Dans cette variante, au lieu de s'étendre jusqu'à l'extrémité distale de l'âme centrale 50, les deux couches PZT 46, 48 sont plus courtes et ne sont pas recouvertes par les demi-masses inertielles 26a, 26b. L'avantage de cette configuration est de favoriser l'espace disponible pour l'amplitude des masses lors du débattement du sous-ensemble. L'espace disponible est ainsi optimisé en terme de masse volumique embarquée et de déflexion de l'ensemble. Le gain de volume, typiquement de 10% environ, peut être reporté sur les masses inertielles ou bien sur le débattement du sous-ensemble (typiquement de 0,5 mm), ce qui permet d'augmenter les performances électriques du système récupérateur d'énergie.

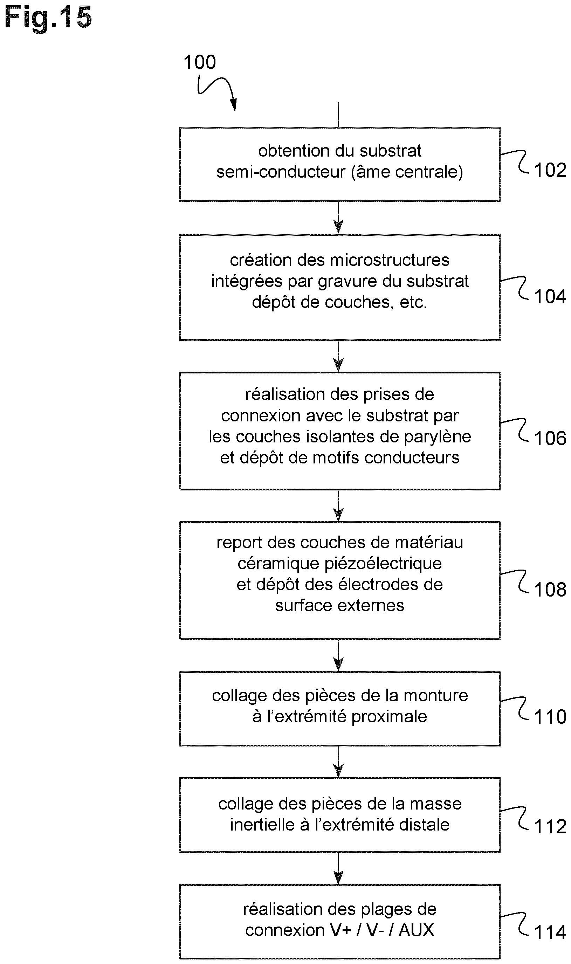

La Figure 15 est un organigramme présentant les différentes étapes d'un processus de fabrication d'un PEH selon l'invention tel que celui que l'on vient de décrire.

La première étape (bloc 102 de l'organigramme 100) de la Figure 12 consiste à obtenir l'âme centrale 50 à partir d'un matériau semi-conducteur (typiquement le silicium) apte à former un substrat de circuit intégré.

L'étape suivante (bloc 104) consiste à créer les microstructures intégrées par gravure du substrat semi-conducteur, dépôt de couches conductrices ou isolantes, etc., selon des techniques de fabrication bien connues en elles-mêmes, utilisées sans adaptation particulière et que l'on ne décrira pas en détail.

L'étape suivante (bloc 106) consiste à déposer les couches isolantes intercalaires de parylène 66, 68. Ces couches, dont la surface aura été gravée de manière à définir des pistes électriques avec en épargne des zones électriquement non conductrices, réalisent les prises de connexion avec le substrat et les interconnexions entre les différentes microstructures.

L'étape suivante (bloc 108) consiste à reporter sur l'ensemble formé par l'âme centrale 50 et les couches intercalaires 66, 68 les couches de matériau céramique piézoélectrique 46, 48, sur lesquelles seront déposées les électrodes de surface 52, 54 pour le recueil des charges électriques.

L'étape suivante (bloc 110) consiste à coller à l'extrémité proximale (correspondant à l'extrémité encastrée) de la lame ainsi obtenue les éléments 24a, 24b de la monture.

L'étape suivante (bloc 112) consiste à coller à l'extrémité distale (correspondant à l'extrémité libre, oscillante) de la lame les deux moitiés 26a, 26b de la masse inertielle.

L'étape finale (bloc 114) consiste à matérialiser côté proximal de la lame les plages de connexion 60, 62, 64 qui pourront être ultérieurement raccordées aux circuits électriques du dispositif à alimenter par le PEH ainsi constitué.

L'ensemble se présente alors dans son état assemblé final, tel qu'illustré Figure 9.

## Revendications

1. Un récupérateur d'énergie à transducteur piézoélectrique, PEH, (40) comprenant un ensemble pendulaire soumis à des sollicitations externes appliquées au récupérateur, l'ensemble pendulaire comprenant :
- une lame (22) élastiquement déformable en flexion ;
- une monture de lame (24), couplée avec encastrement à une extrémité proximale de la lame (22) ; et
- une masse inertielle (26), montée à une extrémité distale, libre, de la lame (22),
dans lequel la lame (22) est une lame piézoélectrique apte à convertir en un signal électrique oscillant (V_{OUT}(t)) une énergie mécanique produite par des oscillations de l'ensemble pendulaire,
et dans lequel la lame piézoélectrique (22) comprend un structure sandwich flexible comportant :
. une âme centrale (50) ;
. sur au moins l'une des faces de l'âme centrale (50), une couche piézoélectrique (46, 48) ; et
. sur une face externe de la couche piézoélectrique (46, 48), au moins une électrode de surface (52, 54), l'âme centrale (50) de la structure sandwich flexible étant en un matériau semi-conducteur apte à former un substrat de circuit intégré et le substrat en matériau semi-conducteur de l'âme centrale (50) de la structure sandwich flexible comportant des structures intégrées monolithiques, **caractérisé en ce que** l'agencement, sur l'étendue du substrat de l'âme centrale (50), desdites structures intégrées monolithiques forme en direction longitudinale une pluralité de zones successives (z_{d}, z_{c}, zₚ) ayant des coefficients de raideur en flexion de la lame piézoélectrique (22) différents d'une zone à l'autre.

2. Le PEH. (40) de la revendication 1, dans lequel lesdites structures intégrées monolithiques sont des composants électriques ou électroniques intégrés (42, 44, 56, 58).

3. Le PEH (40) de la revendication 2, dans lequel lesdits composants électriques ou électroniques intégrés sont :
des composants électroniques d'une unité de gestion d'alimentation, PMU, (42) intégrée à l'âme centrale (50) de la structure sandwich flexible, et/ou
des composants *solid-state* d'un organe de stockage d'énergie (44) intégré à l'âme centrale (50) de la structure sandwich flexible, et/ou
des composants électroniques d'un processeur numérique intégré à l'âme centrale (50) de la structure sandwich flexible.

4. Le PEH (40) de la revendication 1, dans lequel la pluralité de zones successives comprend, en direction longitudinale, au moins une zone centrale (z_{c}) et des zones distale (z_{d}) et proximale (zₚ) attenantes, et dans lequel la raideur en flexion de la zone centrale (z_{c}) est inférieure à la raideur en flexion des zones distale et proximale (z_{d}, zₚ) attenantes.

5. Le PEH (40) de la revendication 3, dans lequel la zone distale (z_{d}) est au moins partiellement située dans une région de la masse inertielle (26).

6. Le PEH (40) de la revendication 3, dans lequel la zone proximale (zₚ) est au moins partiellement située dans une région de la monture de lame (24).

7. Un dispositif autonome logeant, dans un corps de dispositif :
- un ensemble électronique (28-38) ;
- un organe de stockage d'énergie (44) ; et
- un PEH (40) selon l'une des revendications 1 à 6, pour l'alimentation de l'ensemble électronique (28-38) et/ou la recharge de l'organe de stockage d'énergie (44).

8. Le dispositif autonome de la revendication 7,
dans lequel le dispositif autonome est un dispositif médical actif de type capsule autonome implantable (10) comprenant un corps de capsule (12) avec un élément (16) d'ancrage à une paroi d'un organe d'un patient,
et dans lequel les sollicitations externes auxquelles est soumis le PEH (40) sont des sollicitations appliquées au corps de capsule (12) sous l'effet de mouvements de ladite paroi et/ou de variations de débit d'un flux dans un milieu environnant.

9. Un procédé de fabrication d'un récupérateur d'énergie à transducteur piézoélectrique, PEH, (40) comprenant les étapes successives suivantes :
a) obtention d'une âme centrale (50) en un matériau semi-conducteur apte à former un substrat de circuit intégré ;
b) intégration de structures intégrées monolithiques dans le substrat en matériau semi-conducteur de l'âme centrale (50)
c) dépôt, sur au moins l'une des faces de l'âme centrale (50), d'une couche piézoélectrique (46, 48) ; et
d) dépôt d'au moins une électrode de surface (52, 54) sur une face externe de la couche piézoélectrique (46, 48),
**caractérisé en ce que** l'agencement, sur l'étendue du substrat de l'âme centrale (50), desdites structures intégrées monolithiques forme en direction longitudinale une pluralité de zones successives (z_{d}, z_{c}, zₚ) ayant des coefficients de raideur en flexion de la lame piézoélectrique (22) différents d'une zone à l'autre.

10. Le procédé de la revendication 9,
dans lequel l'étape b) comprend l'intégration sous forme monolithique de composants électriques ou électroniques (42, 44, 56, 58).

11. Le procédé de la revendication 10, dans lequel lesdits composants électriques ou électroniques intégrés à l'étape b) sont :
des composants électroniques d'une unité de gestion d'alimentation, PMU, (42) intégrée à l'âme centrale (50) de la structure sandwich flexible, et/ou
des composants *solid-state* d'un organe de stockage d'énergie (44) intégré à l'âme centrale (50) de la structure sandwich flexible, et/ou
des composants électroniques d'un processeur numérique intégré à l'âme centrale (50) de la structure sandwich flexible.

12. Le procédé de la revendication 9, comprenant en outre des étapes ultérieures de :
e) montage d'une masse inertielle (26) à une extrémité distale, libre, de la lame (22) ; et
f) couplage avec encastrement d'une extrémité proximale de la lame (22) à une monture de lame (24).

13. Le procédé de la revendication 12, dans lequel l'étape e) comprend le collage de demi-masses (26a, 26b) directement sur les faces externes respectives de la couche piézoélectrique (46, 48), et/ou l'étape f) comprend le collage de deux éléments de monture (24a, 24b) directement sur les faces externes respectives de la couche piézoélectrique (46, 48).

## Patentansprüche

1. Eine Vorrichtung zur Energiegewinnung über einen piezoelektrischen Wandler, PEH, (40), umfassend eine Pendelanordnung, die äußere Belastungen erfährt, die auf die Vorrichtung zur Energiegewinnung aufgebracht werden, wobei die Pendelanordnung Folgendes umfasst:
- eine durch Biegung elastisch verformbare Zunge (22);
- eine Zungenhalterung (24), die an ein proximales Ende der Zunge (22) formschlüssig gekoppelt ist; und
- eine inertiale Masse (26), die an einem distalen, freien, Ende der Zunge (22) montiert ist,
wobei die Zunge (22) eine piezoelektrische Zunge ist, die imstande ist, eine von Schwingungen der Pendelanordnung erzeugte mechanische Energie in ein elektrisches Schwingungssignal (V_{OUT}(t)) umzuwandeln, und wobei die piezoelektrische Zunge (22) eine flexible Sandwich-Struktur umfasst, mit:
· einem Mittelkern (50);
· auf wenigstens einer der Flächen des Mittelkerns (50), einer piezoelektrischen Schicht (46, 48); und,
· auf einer äußeren Fläche der piezoelektrische Schicht (46, 48), wenigstens einer Oberflächenelektrode (52, 54),
wobei der Mittelkern (50) der flexiblen Sandwich-Struktur aus einem Halbleitermaterial ist, das imstande ist, ein Substrat für eine integrierte Schaltung zu bilden, und das Substrat aus Halbleitermaterial des Mittelkerns (50) der flexiblen Sandwich-Struktur integrierte monolithische Strukturen aufweist,
**dadurch gekennzeichnet, dass** die Anordnung, über die räumliche Ausdehnung des Substrats des Mittelkerns (50), der integrierten monolithischen Strukturen in Längsrichtung eine Mehrzahl aufeinander folgender Zonen (z_{d}, z_{c}, zₚ) bildet, die Biegesteifigkeitskoeffizienten der piezoelektrischen Zunge (22) aufweisen, die von einer Zone zur anderen verschieden sind.

2. Die PEH (40) nach Anspruch 1, wobei die integrierten monolithischen Strukturen integrierte elektrische oder elektronische Bauteile (42, 44, 56, 58) sind.

3. Die PEH (40) nach Anspruch 2, wobei die integrierten elektrischen oder elektronischen Bauteile Folgendes sind:
elektronische Bauteile einer Leistungsverwaltungseinheit, PMU, (42), die in den Mittelkern (50) der flexiblen Sandwich-Struktur integriert ist, und/oder
Solid-State-Bauteile eines Energiespeicherorgans (44), das in den Mittelkern (50) der flexiblen Sandwich-Struktur integriert ist,
und/oder elektronische Bauteile eines Digitalprozessors, der in den Mittelkern (50) der flexiblen Sandwich-Struktur integriert ist.

4. Die PEH (40) nach Anspruch 1, wobei die Mehrzahl aufeinander folgender Zonen, in Längsrichtung, wenigstens eine zentrale Zone (z_{c}), eine distale Zone (z_{d}) und eine proximale Zone (zₚ), die sich anschließen, umfasst, und wobei die Biegesteifigkeit der zentralen Zone (z_{c}) geringer ist als die Biegesteifigkeit der distalen Zone (z_{d}) und der proximalen Zone (zₚ), die sich anschließen.

5. Die PEH (40) nach Anspruch 3, wobei die distale Zone (z_{d}) sich wenigstens teilweise in einer Region der inertialen Masse (26) befindet.

6. Die PEH (40) nach Anspruch 3, wobei die proximale Zone (zₚ) sich wenigstens teilweise in einer Region der Zungenhalterung (24) befindet.

7. Eine autonome Vorrichtung, die in einem Vorrichtungskörper Folgendes aufnimmt:
- eine elektronische Anordnung (28-38);
- ein Energiespeicherorgan (44); und
- eine PEH (40) nach einem der Ansprüche 1 bis 6 für die Versorgung der elektronischen Anordnung (28-38) und/oder für die Wiederaufladung des Energiespeicherorgans (44).

8. Die autonome Vorrichtung nach Anspruch 7,
wobei die autonome Vorrichtung eine aktive medizinische Vorrichtung (10) vom Typ implantierbare autonome Kapsel ist, umfassend einen Kapselkörper (12) mit einem Element (16) zur Verankerung an einer Wand eines Organs eines Patienten,
und wobei die äußeren Belastungen, die die PEH (40) erfährt, Belastungen sind, die auf den Kapselkörper (12) unter der Einwirkung von Bewegungen der Wand und/oder von Veränderungen einer Durchflussrate in einem umgebenden Medium aufgebracht werden.

9. Ein Verfahren zur Herstellung einer Vorrichtung zur Energiegewinnung über einen piezoelektrischen Wandler, PEH, (40), umfassend die folgenden aufeinander folgenden Schritte:
a) Bereitstellen eines Mittelkerns (50) aus einem Halbleitermaterial, das imstande ist, ein Substrat für eine integrierte Schaltung zu bilden;
b) Integrieren von integrierten monolithischen Strukturen in das Substrat aus Halbleitermaterial des Mittelkerns (50);
c) Aufbringen, auf wenigstens einer der Flächen des Mittelkerns (50), einer piezoelektrischen Schicht (46, 48); und
d) Aufbringen wenigstens einer Oberflächenelektrode (52, 54) auf einer äußeren Fläche der piezoelektrischen Schicht (46, 48),
**dadurch gekennzeichnet, dass** die Anordnung, über die räumliche Ausdehnung des Substrats des Mittelkerns (50), der integrierten monolithischen Strukturen in Längsrichtung eine Mehrzahl aufeinander folgender Zonen (z_{d}, z_{c}, zₚ) bildet, die Biegesteifigkeitskoeffizienten der piezoelektrischen Zunge (22) aufweisen, die von einer Zone zur anderen verschieden sind.

10. Das Verfahren nach Anspruch 9,
wobei Schritt b) das Integrieren in monolithischer Form von elektrischen oder elektronischen Bauteilen (42, 44, 56, 58) umfasst.

11. Das Verfahren nach Anspruch 10, wobei die in Schritt b) integrierten elektrischen oder elektronischen Bauteile Folgendes sind:
elektronische Bauteile einer Leistungsverwaltungseinheit (PMU) (42), die in den Mittelkern (50) der flexiblen Sandwich-Struktur integriert ist, und/oder
Solid-State-Bauteile eines Energiespeicherorgans (44), das in den Mittelkern (50) der flexiblen Sandwich-Struktur integriert ist, und/oder
elektronische Bauteile eines Digitalprozessors, der in den Mittelkern (50) der flexiblen Sandwich-Struktur integriert ist.

12. Das Verfahren nach Anspruch 9, ferner umfassend folgende weitere Schritte:
e) Montieren einer inertialen Masse (26) an einem distalen, freien, Ende der Zunge (22); und
f) formschlüssiges Koppeln eines proximalen Endes der Zunge (22) an einer Zungenhalterung (24).

13. Das Verfahren nach Anspruch 12, wobei Schritt e) das Aufkleben von Halbmassen (26a, 26b) direkt auf den jeweiligen äußeren Flächen der piezoelektrischen Schicht (46, 48) umfasst und/oder Schritt f) das Aufkleben von zwei Halterungselementen (24a, 24b) direkt auf den jeweiligen äußeren Flächen der piezoelektrischen Schicht (46, 48) umfasst.

## Claims

1. A piezoelectric-transducer energy harvester, PEH, (40) comprising a pendular unit subjected to external stresses applied to the harvester, the pendular unit comprising:
- a beam (22) that is elastically deformable in bending;
- a beam mount (24) with clamped coupling to a proximal end of the beam (22); and
- an inertial mass (26), mounted at a free, distal end of the beam (22),
wherein the beam (22) is a piezoelectric beam adapted to convert into an oscillating electric signal (V_{OUT}(t)) a mechanical energy produced by oscillations of the pendular unit,
and wherein the piezoelectric beam (22) comprises a flexible sandwiched structure including:
. a central core (50);
. a piezoelectric layer (46, 48) on at least one face of the central core (50); and
. at least one surface electrode (52, 54) on an external face of the piezoelectric layer (46, 48),
wherein the central core (50) of the flexible sandwiched structure is made of a semiconductor material adapted to form an integrated circuit substrate and the substrate in a semiconductor material of the central core (50) of the flexible sandwiched structure includes monolithic integrated structures,
**characterized in that** the arrangement, over the extend of the substrate of the central core (50), of said monolithic integrated structures forms in a longitudinal direction a plurality of successive areas (z_{d}, z_{c}, zₚ) having different piezoelectric beam bending stiffness coefficients from an area to another.

2. The PEH (40) of claim 1, wherein said monolithic integrated structures are integrated electric or electronic components (42, 44, 56, 58).

3. The PEH (40) of claim 2, wherein said integrated electric or electronic components are:
electronic components of a power management unit, PMU, (42) integrated to the central core (50) of the flexible sandwiched structure, and/or
solid-state components of an energy storage element (44) integrated to the central core (50) of the flexible sandwiched structure, and/or
electronic components of a digital processor integrated to the central core (50) of the flexible sandwiched structure.

4. The PEH (40) of claim 1, wherein the plurality of successive areas comprises, in a longitudinal direction, at least one central area (z_{c}) and adjoining distal (z_{d}) and proximal (zₚ) areas, and wherein the bending stiffness of the central area (z_{c}) is lower than the bending stiffness of the adjoining distal (z_{d}) and proximal (zₚ) areas.

5. The PEH (40) of claim 4, wherein the distal area (z_{d}) is at least partially located in a region of the inertial mass (26).

6. The PEH (40) of claim 4, wherein the proximal area (zₚ) is at least partially located in a region of the beam mount (24).

7. An autonomous device housing, within a device body:
- an electronic unit (28-38);
- an energy storage element (44); and
- a PEH (40) according one of claims 1 to 6, for powering the electronic unit (28-38) and/or charging the energy storage element (44).

8. The autonomous device of claim 7,
wherein the autonomous device is an active medical device of the implantable autonomous capsule type (10) comprising a capsule body (12) with an element (16) for its anchoring to a wall of a patient's organ,
and wherein the external stresses to which is subjected the PEH (40) are stresses applied to the capsule body (12) under the effect of movements of said wall and/or flow rate variations of a flow in a surrounding environment.

9. A method for manufacturing a piezoelectric-transducer energy harvester, PEH, (40) comprising the following successive steps:
a) obtaining a central core (50) made of a semiconductor material, adapted to form an integrated circuit substrate;
b) integrating monolithic structures in the semiconductor material substrate of the central core (50);
c) depositing a piezoelectric layer (46, 48) on at least one face of the central core (50); and
d) depositing at least one surface electrode (52, 54) on an external face of the piezoelectric layer (46, 48),
**characterized in that** the arrangement, over the extend of the substrate of the central core (50), of said monolithic integrated structures forms in a longitudinal direction a plurality of successive areas (z_{d}, z_{c}, zₚ) having different piezoelectric beam bending stiffness coefficients from an area to another.

10. The method of claim 9,
wherein step b) comprises the integration in monolithic form of electric or electronic components (42, 44, 56, 58).

11. The method of claim 10, wherein said electric or electronic components integrated at step b) are:
electronic components of a power management unit, PMU, (42) integrated to the central core (50) of the flexible sandwiched structure, and/or
solid-state components of an energy storage element (44) integrated to the central core (50) of the flexible sandwiched structure, and/or
electronic components of a digital processor integrated to the central core (50) of the flexible sandwiched structure.

12. The method of claim 9, further comprising subsequent steps of:
e) mounting an inertial mass (26) at a free, distal end of the beam (22); and
f) clamped coupling a proximal end of the beam (22) in a beam mount (24).

13. The method of claim 12, wherein step e) comprises gluing half-masses (26a, 26b) directly on respective external faces of the piezoelectric layer (46, 48), and/or step f) comprises gluing two mount elements (24a, 24b) directly on respective external faces of the piezoelectric layer (46, 48).
